# EUROPÄISCHE PATENTANMELDUNG

(11) **EP 1 486 564 A1**
(43) Veröffentlichungstag der Anmeldung: **15.12.2004**
(21) Anmeldenummer: 03013296.3
(22) Anmeldetag: 13.06.2003
(51) Int. Cl.: C12N 15/11, A61K 31/713

(54) **SiRNA mit erhöhter Stabilität in Serum**

(71) Anmelder: Ribopharma AG, 95326 Kulmbach (DE)
(72) Erfinder: Vornlocher, Hans-Peter, Dr., 95448 Bayreuth (DE); John, Matthias, Dr., 96103 Hallstadt (DE); Hadwiger, Philipp, Dr., 95448 Bayreuth (DE); Woppmann, Claudia, 95494 Gesees (DE); Kreutzer, Roland, Dr., 95466 Weidenberg (DE); Limmer, Stefan, Dr., 95326 Kulmbach (DE)
(74) Vertreter: Gassner, Wolfgang, Dr.

(57) **Zusammenfassung**

Die Erfindung betrifft ein Verfahren zur gezielten Auswahl einer eine erhöhte Wirksamkeit aufweisenden doppelsträngigen aus zwei Einzelsträngen bestehenden Ribonukleinsäure (dsRNA) zur Hemmung der Expression eines Zielgens durch RNA-Interferenz, wobei die Sequenzen der Einzelstränge der dsRNA so gewählt werden, dass an beiden Enden der dsRNA jeweils das letzte komplementäre Nukleotidpaar G-C ist oder mindestens zwei der letzten vier komplementären Nukleotidpaare G-C-Paare sind, wobei die dsRNA am ersten Ende einen aus 1 bis 4 ungepaarten Nukleotiden gebildeten einzelsträngigen Überhang aufweist und am zweiten Ende keinen Überhang aufweist, wobei das sich direkt an das letzte komplementäre Nukleotidpaar anschließende ungepaarte Nukleotid des einzelsträngigen Übergangs eine Purin-Base enthält.

SEQUENZPROTOKOLL

<110>  Ribopharma AG

<120>  Doppelsträngige Ribonukleinsäure mit erhöhter Wirksamkeit in einem
Organismus

<130>  432724EH

<160>  16

<170>  PatentIn version 3.1

<210>  1

<211>  22

<212>  RNA

<213>  Künstliche Sequenz

<220>

<223>  Sinn-Strang einer dsRNA, deren Antisinn-Strang (SEQ ID NO: 2) zu
einer Sequenz aus dem Sinn-Strang des humanen Bcl-2-Gens komplementär
ist.

<400>  1
caggaccucg ccgcugcaga cc                                              22

<210>  2

<211>  24

<212>  RNA

<213>  Künstliche Sequenz

<220>

<223>  Antisinn-Strang einer dsRNA, der zu einer Sequenz aus dem Sinn-
Strang des humanen Bcl-2-Gens komplementär ist.

<400>  2
ggucugcagc ggcgaggucc uggc                                            24

<210>  3

<211>  22

<212>  RNA

<213>  Künstliche Sequenz

<220>

<223>  Sinn-Strang einer dsRNA, deren Antisinn-Strang (SEQ ID NO: 4) zu
einer Sequenz aus dem Sinn-Strang des humanen Bcl-2-Gens komplementär
ist.

<400>  3
gccuuugugg aacuguacgg cc                                                22

<210>  4

<211>  24

<212>  RNA

<213>  Künstliche Sequenz

<220>

<223>  Antisinn-Strang einer dsRNA, der zu einer Sequenz aus dem Sinn-
Strang des humanen Bcl-2-Gens komplementär ist.

<400>  4
ggccguacag uuccacaaag gcau                                              24

<210>  5

<211>  24

<212>  RNA

<213>  Künstliche Sequenz

<220>

<223>  Sinn-Strang einer dsRNA, deren Antisinn-Strang (SEQ ID NO: 6) zu
einer Sequenz aus dem Sinn-Strang des Proteinkinase C-Gens komplementär
ist.

<400>  5
cuucuccgcc ucacaccgcu gcaa                                              24

<210>  6

<211>  22

<212>  RNA

<213>  Künstliche Sequenz

<220>

<223>  Antisinn-Strang einer dsRNA, der zu einer Sequenz aus dem Sinn-
Strang des Proteinkinase C-Gens komplementär ist.

<400>  6
gcagcggugu gaggcggaga ag                                                22


<210>  7

<211>  21

<212>  RNA

<213>  Künstliche Sequenz



<220>

<223> Sinn-Strang einer dsRNA, deren Antisinn-Strang (SEQ ID NO: 8) zu
einer Sequenz aus dem Sinn-Strang des humanen Bcl-2-Gens komplementär
ist.

<400>  7
ggcgacuucg ccgagauguc c                                                 21


<210>  8

<211>  23

<212>  RNA

<213>  Künstliche Sequenz



<220>

<223>  Antisinn-Strang einer dsRNA, der zu einer Sequenz aus dem
Sinn-Strang des humanen bcl-2-Gens komplementär ist.

<400>  8
ggacaucucg gcgaagucgc cgc                                               23


<210>  9

<211>  21

<212>  RNA

<213>  Künstliche Sequenz

<220>

<223> Sinn-Strang einer dsRNA, deren Antisinn-Strang (SEQ ID NO: 10) zu
einer Sequenz aus dem Sinn-Strang des humanen bcl-2-Gens (GenBank, Ein-
tragungsnummer M13994) komplementär ist.

<400> 9
accgggcauc uucuccuccc a                                                21


<210> 10

<211> 23

<212> RNA

<213> Künstliche Sequenz



<220>

<223> Antisinn-Strang einer dsRNA, der zu einer Sequenz aus dem Sinn-
Strang des humanen bcl-2-Gens (GenBank, Eintragungsnummer M13994) komple-
mentär ist.

<400> 10
ugggaggaga agaugcccgg ugc                                              23


<210> 11

<211> 21

<212> RNA

<213> Künstliche Sequenz



<220>

<223> Sinn-Strang einer dsRNA, deren Antisinn-Strang (SEQ ID NO: 12) zu
einer Sequenz aus dem Sinn-Strang des humanen PLK1-Gens (GenBank, Eintra-
gungsnummer X75932) komplementär ist.

<400> 11
gaucacccuc cuuaaauauu u                                                21


<210> 12

<211> 23

<212> RNA

<213> Künstliche Sequenz


<220>

<223>  Antisinn-Strang einer dsRNA, der zu einer Sequenz aus dem Sinn-
Strang des humanen PLK1-Gens (GenBank, Eintragungsnummer X75932) komple-
mentär ist.

<400>  12
aaauauuuaa ggagggugau cgc                                              23

<210>  13

<211>  21

<212>  RNA

<213>  Hepatitis C Virus

<400>  13
acggcuagcu gugaaagguc c                                                21

<210>  14

<211>  23

<212>  RNA

<213>  Hepatitis C Virus

<400>  14
ggaccuuuca cagcuagccg uga                                              23

<210>  15

<211>  21

<212>  RNA

<213>  Homo sapiens

<400>  15
caaggagcag ggacaaguua c                                                21

<210>  16

<211>  23

<212>  RNA

<213>  Homo sapiens

<400>  16
guaacuuguc ccugcuccuu gaa                                              23

## Beschreibung

Die Erfindung betrifft eine doppelsträngige aus zwei Einzelsträngen bestehende Ribonukleinsäure (dsRNA) mit erhöhter Wirksamkeit in einem Organismus, ein Verfahren zu deren gezielter Auswahl, ein diese dsRNA enthaltendes Medikament sowie eine Verwendung einer solchen doppelsträngigen Ribonukleinsäure.

Es ist bekannt, dsRNA zur Hemmung der Expression eines Zielgens durch RNA-Interferenz einzusetzen. Bisher eingesetzte dsRNA war dabei von unterschiedlicher Wirksamkeit. Aus der WO 02/44321 A2 ist ein Zusammenhang zwischen der Wirksamkeit einer dsRNA, deren Länge und der Position und Länge von Überhängen aus ungepaarten Nukleotiden bekannt. Bei einem zwei Nukleotide langen Überhang am 3'-Ende eines Strangs der dsRNA wurde gefunden, dass es für die Effizienz besonders günstig ist, wenn das vorletzte Nukleotid am 3'-Ende ein U ist.

Aufgabe der vorliegenden Erfindung ist es, ein Verfahren zur gezielten Auswahl einer eine erhöhte Wirksamkeit in einem Organismus aufweisenden dsRNA zur Hemmung der Expression eines Zielgens durch RNA-Interferenz, eine Verwendung einer solchen dsRNA, eine solche dsRNA und ein eine solche dsRNA enthaltendes Medikament bereitzustellen.

Die Aufgabe wird durch die Merkmale der Ansprüche 1, 9, 17 und 18 gelöst. Zweckmäßige Ausgestaltungen ergeben sich aus den Merkmalen der Ansprüche 2 bis 8 und 10 bis 16.

Erfindungsgemäß ist ein Verfahren zur gezielten Auswahl einer eine erhöhte Wirksamkeit aufweisenden doppelsträngigen aus zwei Einzelsträngen bestehenden Ribonukleinsäure (dsRNA) zur Hemmung der Expression eines Zielgens durch RNA-Interferenz vorgesehen, wobei die Sequenzen der Einzelstränge der dsRNA so gewählt werden, dass an beiden Enden der dsRNA jeweils das letzte komplementäre Nukleotidpaar G-C ist oder mindestens zwei der letzten vier komplementären Nukleotidpaare G-C-Paare sind, wobei die dsRNA am ersten Ende einen aus 1 bis 4 ungepaarten Nukleotiden gebildeten einzelsträngigen Überhang aufweist und am zweiten Ende keinen Überhang aufweist, wobei das sich direkt an das letzte komplementäre Nukleotidpaar anschließende ungepaarte Nukleotid des einzelsträngigen Übergangs eine Purin-Base enthält, wobei die folgenden dsRNAs ausgenommen sind: 
5'-   CAG GAC CUC GCC GCU GCA GAC C-3' (SEQ ID NO: 1)
3'-CG GUC CUG GAG CGG CGA CGU CUG G-5' (SEQ ID NO: 2),
 
5'- G CCU UUG UGG AAC UGU ACG GCC-3' (SEQ ID NO: 3)
3'-UAC GGA AAC ACC UUG ACA UGC CGG-5' (SEQ ID NO: 4),
 
5'-CUU CUC CGC CUC ACA CCG CUG CAA-3' (SEQ ID NO: 5)
3'-GAA GAG GCG GAG UGU GGC GAC G  -5' (SEQ ID NO: 6),
 
5'-  A CGG CUA GCU GUG AAA GGU CC-3'  (SEQ ID NO: 13)
3'-AGU GCC GAU CGA CAC UUU CCA GG-5'  (SEQ ID NO: 14)
 und

5'-   CAA GGA GCA GGG ACA AGU UAC-3' (SEQ ID NO: 15)
3'-AA GUU CCU CGU CCC UGU UCA AUG-5' (SEQ ID NO: 16).

"G" steht für ein Guanin, "C" für ein Cytosin, "A" für ein Adenin und "U" für ein Uracil jeweils als Base enthaltendes Nukleotid. Das "Verfahren zur gezielten Auswahl" umfasst auch ein "Verfahren zum gezielten Auffinden" und ein "Verfahren zum gezielten Herstellen". Unter dem "Zielgen" wird im Allgemeinen der Abschnitt eines DNA-Strangs der doppelsträngigen DNA in der Zelle verstanden, welcher komplementär zu einem Abschnitt des anderen DNA-Strangs der doppelsträngigen DNA ist, der bei der Transkription als Matrize dient. Der Abschnitt umfasst dabei alle transkribierten Bereiche. Bei dem Zielgen handelt es sich also im Allgemeinen um den Sinn-Strang. Ein Antisinn-Strang der dsRNA kann somit komplementär zu einem bei der Expression des Zielgens gebildeten RNA-Transkript oder dessen Prozessierungsprodukt, wie z.B. einer mRNA, sein. Ein Sinn-Strang der dsRNA ist der zum Antisinn-Strang komplementäre Strang der dsRNA. Eine dsRNA liegt vor, wenn die aus zwei Ribonukleinsäure-Strängen bestehende Ribonukleinsäure eine doppelsträngige Struktur aufweist. Nicht alle Nukleotide der dsRNA müssen Watson-Crick-Basenpaarungen aufweisen. Insbesondere einzelne nicht komplementäre Basenpaare beeinträchtigen die Wirkung der dsRNA bei der Expressionshemmung durch RNA-Interferenz kaum oder gar nicht.

Die Sequenzen der Einzelstränge der dsRNA können gewählt werden, indem innerhalb des zu hemmenden Zielgens ein Bereich und dessen Länge so gewählt wird, dass eine dsRNA mit einem dazu komplementären Strang die oben genannten Merkmale aufweist. Da einzelne nicht zum Zielgen komplementäre Nukleotide die RNA-Interferenz nicht verhindern, ist es auch möglich, an den zum Zielgen komplementären Bereich eines Strangs der dsRNA ein einzelnes oder einzelne Nukleotide anzuhängen oder einzelne Nukleotide in dem Strang zu ersetzen, um eine dsRNA zu erhalten, welche die erfindungsgemäß definierten Merkmale aufweist.

Bei der WO 02/44321 A2 ist es bei der Untersuchung der Wirksamkeit außer Acht gelassen worden, dass die Wirksamkeit einer dsRNA bei einer medizinischen Anwendung in einem Organismus auch von deren Bioverfügbarkeit abhängig ist. Diese ist umso höher, je stabiler die dsRNA im Blut ist und je länger sie somit verfügbar ist. Die Stabilität von dsRNA im Blut wird vor allem durch deren Abbaubarkeit durch im Blut vorhandene Enzyme bestimmt. Überraschenderweise hat sich gezeigt, dass diese Abbaubarkeit von den Sequenzen der die dsRNA bildenden Einzelstränge abhängig ist. Durch das erfindungsgemäße Verfahren wird eine dsRNA mit erhöhter Stabilität im Blut und somit einer höheren Bioverfügbarkeit als eine andere dsRNA ausgewählt. Da die Stabilität in Blut experimentell näherungsweise gemessen wird, indem man die Stabilität im Serum, also in der von zellulären Bestandteilen und Gerinnungsfaktoren befreiten wässrigen Phase des Blutes, bestimmt, wird diese Stabilität im Folgenden als Serumstabilität bezeichnet. Diese Formulierung ist jedoch in keiner Weise limitierend.

Die Formulierung "eine doppelsträngige aus zwei Einzelsträngen bestehende Ribonukleinsäure", bedeutet hier, dass es sich nicht um einen teilweise selbst-assoziierten Einzelstrang (stem-loop) handelt. Eine Verknüpfung zwischen den Einzelsträngen ist durch diese Formulierung jedoch nicht ausgeschlossen. Die beiden Ribonukleinsäure-Einzelstränge können, z.B. über eine oder vorzugsweise mehrere chemische Verknüpfungen, verbunden sein. Dadurch kann die Stabilität der dsRNA weiter erhöht werden. Beispielsweise kann das 5'-Ende des Antisinn-Strangs über einen Hexa-ethylenglycol-Linker mit dem 3'-Ende des Sinn-Strangs verbunden sein. Dem Fachmann sind viele Möglichkeiten einer solchen Verbindung zur weiteren Stabilisierung der dsRNA bekannt.

Die chemische Verknüpfung wird zweckmäßigerweise durch eine kovalente oder ionische Bindung, eine Wasserstoffbrückenbindung, hydrophobe Wechselwirkungen, vorzugsweise van-der-Waals- oder Stapelungswechselwirkungen, oder durch Metall-Ionenkoordination gebildet. Sie kann nach einem besonders vorteilhaften Ausgestaltungsmerkmal an mindestens einem, vorzugsweise an beiden, Ende/n hergestellt werden.

Es hat sich weiter als vorteilhaft erwiesen, daß die chemische Verknüpfung mittels einer oder mehrerer Verbindungsgruppen gebildet wird, wobei die Verbindungsgruppen vorzugsweise Poly-(oxyphosphinicooxy-1,3-propandiol)- und/oder Polyethylenglycol-Ketten sind. Die chemische Verknüpfung kann auch durch in der doppelsträngigen Struktur anstelle von Purinen benutzte Purinanaloga gebildet werden. Von Vorteil ist es ferner, daß die chemische Verknüpfung durch in der doppelsträngigen Struktur eingeführte Azabenzoleinheiten gebildet wird. Sie kann außerdem durch in der doppelsträngigen Struktur anstelle von Nukleotiden benutzte verzweigte Nukleotidanaloga gebildet werden.

Es hat sich als zweckmäßig erwiesen, dass zur Herstellung der chemischen Verknüpfung mindestens eine der folgenden Gruppen benutzt wird: Methylenblau; bifunktionelle Gruppen, vorzugsweise Bis-(2-chlorethyl)-amin; N-acetyl-N'-(p-glyoxyl-benzoyl)-cystamin; 4-Thiouracil; Psoralen. Ferner kann die chemische Verknüpfung durch an den Enden des doppelsträngigen Bereichs angebrachte Thiophosphoryl-Gruppen gebildet werden. Vorzugsweise wird die chemische Verknüpfung an den Enden des doppelsträngigen Bereichs durch Tripelhelix-Bindungen hergestellt.

Die chemische Verknüpfung kann zweckmäßigerweise durch ultraviolettes Licht induziert werden.

Die Nukleotide der dsRNA können modifiziert sein. Dies wirkt einer Aktivierung einer von doppelsträngiger RNA abhängigen Proteinkinase, PKR, in der Zelle entgegen. Vorteilhafterweise ist mindestens eine 2'-Hydroxylgruppe der Nukleotide der dsRNA in der doppelsträngigen Struktur durch eine chemische Gruppe, vorzugsweise eine 2'-Amino- oder eine 2'-Methylgruppe, ersetzt. Mindestens ein Nukleotid in mindestens einem Strang der doppelsträngigen Struktur kann auch ein sogenanntes "locked nucleotide" mit einem, vorzugsweise durch eine 2'-O, 4'-C-Methylenbrücke, chemisch modifizierten Zukkerring sein. Vorteilhafterweise sind mehrere Nukleotide "locked nucleotides".

Vom Schutzumfang ausgenommen sind dsRNAs, die bereits bekannt sind, ohne dass deren Serumstabilität, die damit verbundene verbesserte Wirksamkeit oder ein Zusammenhang zwischen den Sequenzen ihrer Einzelstränge und der Serumstabilität bzw. Wirksamkeit erkannt worden wäre.

Der Vorteil des erfindungsgemäßen Verfahrens besteht darin, dass dadurch eine dsRNA bereitgestellt werden kann, welche in Blut verhältnismäßig stabil ist und durch ihre längere Verfügbarkeit eine höhere Wirksamkeit als andere dsRNAs aufweist. Zur Erhöhung der intrazellulären Wirksamkeit der dsRNA trägt weiterhin bei, dass die dsRNA einen einzelsträngigen Überhang aufweist. Die Stabilität dieser dsRNA wird zusätzlich dadurch erhöht, dass am zweiten Ende der dsRNA kein Überhang vorhanden ist.

Besonders stabil ist die dsRNA im Blut dadurch, dass das sich direkt an das letzte komplementäre Nukleotidpaar anschließende ungepaarte Nukleotid des einzelsträngigen Überhangs eine Purin-Base enthält. Bei der Purin-Base kann es sich um Guanin oder Adenin handeln. Das steht im Widerspruch zur Lehre aus der WO 02/44321 A2, dass es bei einem zwei-Nukleotide langen Überhang am 3'-Ende eines Strangs der dsRNA besonders günstig ist, wenn das vorletzte Nukleotid am 3'-Ende und somit das sich direkt an das letzte komplementäre Nukleotidpaar anschließende ungepaarte Nukleotid ein U, d.h. eine Pyrimidin-Base, ist.

Vorzugsweise besteht der Überhang nur aus einem oder zwei ungepaarten Nukleotiden. Bei einem aus mehr als einem Nukleotid bestehenden einzelsträngigen Überhang wird die Stabilität weiterhin dadurch gesteigert, dass der Überhang zumindest zur Hälfte aus Purin-Basen, insbesondere G oder A, enthaltenden Nukleotiden gebildet ist. Besonders vorteilhaft ist es, wenn der Überhang, insbesondere ausschließlich, die Sequenz 5'-GC-3' aufweist.

Eine noch bessere Wirksamkeit kombiniert mit einer hohen Serumstabilität wird erreicht, wenn das erste Ende der dsRNA am 3'-Ende eines Antisinn-Strangs der dsRNA gelegen ist und das zweite Ende der dsRNA am 3'-Ende eines Sinn-Strangs der dsRNA gelegen ist. Die dsRNA weist dann nur an dem am 3'-Ende des Antisinn-Strangs gelegenen Ende einen einzelsträngigen Überhang auf, während das am 3'-Ende des Sinn-Strangs der dsRNA gelegene Ende der dsRNA keinen Überhang aufweist.

Weiterhin hat es sich als günstig im Bezug auf die Wirksamkeit erwiesen, wenn ein zum Zielgen komplementärer Bereich des Antisinn-Strangs der dsRNA 20 bis 23, insbesondere 22, Nukleotide aufweist. Weiterhin ist es vorteilhaft, wenn der Antisinn-Strang weniger als 30, vorzugsweise weniger als 25, besonders vorzugsweise 21 bis 24, Nukleotide aufweist.

Gegenstand der Erfindung ist außerdem eine doppelsträngige aus zwei Einzelsträngen bestehende Ribonukleinsäure (dsRNA) zur Hemmung der Expression eines Zielgens durch RNA-Interferenz, wobei an beiden Enden der dsRNA jeweils das letzte komplementäre Nukleotidpaar G-C ist oder mindestens zwei der letzten vier komplementären Nukleotidpaare G-C-Paare sind, wobei die dsRNA an einem ersten Ende einen aus 1 bis 4 ungepaarten Nukleotiden gebildeten einzelsträngigen Überhang aufweist und an einem zweiten Ende keinen Überhang aufweist, wobei das sich direkt an das letzte komplementäre Nukleotidpaar anschließende ungepaarte Nukleotid des einzelsträngigen Übergangs eine Purin-Base enthält, wobei die folgenden dsRNAs ausgenommen sind: 
S2:  5'-   CAG GAC CUC GCC GCU GCA GAC C-3' (SEQ ID NO: 1)
S1:  3'-CG GUC CUG GAG CGG CGA CGU CUG G-5' (SEQ ID NO: 2),
 
5'- G CCU UUG UGG AAC UGU ACG GCC-3' (SEQ ID NO: 3)
3'-UAC GGA AAC ACC UUG ACA UGC CGG-5' (SEQ ID NO: 4),
 
5'-CUU CUC CGC CUC ACA CCG CUG CAA-3' (SEQ ID NO: 5)
3'-GAA GAG GCG GAG UGU GGC GAC G  -5' (SEQ ID NO: 6)
 
5'-  A CGG CUA GCU GUG AAA GGU CC-3'  (SEQ ID NO: 13)
3'-AGU GCC GAU CGA CAC UUU CCA GG-5'  (SEQ ID NO: 14)
 und

5'-   CAA GGA GCA GGG ACA AGU UAC-3' (SEQ ID NO: 15)
3'-AA GUU CCU CGU CCC UGU UCA AUG-5' (SEQ ID NO: 16)

Vorteilhafte Ausgestaltungen der dsRNA ergeben sich aus den oben beschriebenen Merkmalen. Weiterhin betrifft die Erfindung die Verwendung einer erfindungsgemäßen dsRNA zur Hemmung der Expression eines Zielgens durch RNA-Interferenz, insbesondere in vitro. Die Erfindung betrifft darüber hinaus ein Medikament zur Hemmung der Expression eines Zielgens durch RNA-Interferenz, wobei das Medikament eine erfindungsgemäße doppelsträngige, aus zwei Einzelsträngen bestehende, dsRNA enthält.

Nachfolgend wird die Erfindung anhand eines Ausführungsbeispiels veranschaulicht. Es zeigen:
- Fig. 1: eine gelelektrophoretische Auftrennung einer erfindungsgemäßen dsRNA ohne und nach 0, 15, 30 , 60, 120 und 240 min Inkubation in Serum,
- Fig. 2: eine gelelektrophoretische Auftrennung einer weiteren erfindungsgemäßen dsRNA ohne und nach 0, 15, 30, 60, 120 und 240 min Inkubation in Serum und
- Fig. 3: eine gelelektrophoretische Auftrennung einer herkömmlichen dsRNA ohne und nach 0, 15, 30, 60, 120 und 240 min Inkubation in Serum.

### RNA Synthese:

Einzelstrang-RNAs wurden durch Festphasensynthese mittels eines Expedite 8909 Synthesizers (Applied Biosystems, Applera Deutschland GmbH, Frankfurter Str. 129b, 64293 Darmstadt, Deutschland) hergestellt. Dazu benötigte Standard Ribonukleosidphosphoramidite und an CPG (Controlled Pore Glass), einem porösen Trägermaterial aus Glas, immobilisierte Nukleoside wurden von der Firma ChemGenes Corporation (Ashiand Technology Center, 200 Homer Avenue, Ashiand, MA 01721, USA) oder der Firma Proligo Biochemie GmbH (Georg-Hyken-Str.14, Hamburg, Deutschland) bezogen. Weitere Synthesereagenzien wurden von der Firma Mallinckrodt Baker (Im Leuschnerpark 4, 64347 Griesheim, Deutschland) bezogen. Rohe Syntheseprodukte wurden mittels HPLC (System Gold, Beckman Coulter GmbH, 85702 Unterschleißheim, Deutschland) über eine Anionenaustauschersäule (DNAPac PA 100, Dionex GmbH, Am Wörtzgarten 10, 65510 Idstein) gereinigt. Die erzielten Ausbeuten wurden durch UV Licht-Absorption bei 260 nm bestimmt.

Die in den Untersuchungen eingesetzten dsRNAs wurden durch 3-minütiges Erhitzen äquimolarer Mengen einzelsträngiger Sinnund Antisinn-RNAs in Annealing-Puffer (100 mM NaCl, 20 mM Na₃PO₄, pH 6,8) auf 90 ± 5°C und langsames Abkühlen auf Raumtemperatur über ca. 3 Stunden hergestellt.

### Gewinnung von humanem Serum:

Eine Blutprobe wurde zur Gerinnung sofort nach ihrer Entnahme in einem abgedunkelten Sammelröhrchen (SST Vacutainer 9,5 ml; BD Vacutainer Systems, Becton Dickinson and Company, Belliver Industrial Estate, Plymouth PL6 7BP, Großbritannien) für 2 Stunden bei 20 °C inkubiert. Danach wurde durch Zentrifugation bei 4°C und 3 000 x g für 15 Minuten (Megafuge 1.0; Heraeus Instruments, Kendro Laboratory Products, D-37520 Osterode, Deutschland) Serum als Überstand von agglutiniertem Blut getrennt, in sterile 1,5 ml Reaktionsgefäße (La Fontaine, International GmbH & Co. KG, Daimlerstr.14, D-68753 Waghäusel, Deutschland) überführt und bei -20°C gelagert.

### Inkubation:

Jeweils 60 µl Serum wurden in 1,5 ml Reaktionsgefäßen auf Eis vorgelegt. Danach wurden jeweils 12 µl einer 25 µM dsRNA-Lösung zugegeben und 5 Sekunden mittels eines Vortex-Genie2 (Scientific Industries Inc., Bohemia, N.Y. 11716, USA) gründlich gemischt. Die dsRNA Konzentration betrug dann 4,16 µM in einem Volumen von 72 µl. Die Proben wurden in einen Heizblock bei 37°C für 15, 30, 60, 120 und 240 Minuten inkubiert und anschließend sofort in flüssigem Stickstoff schockgefroren. Eine Probe wurde ohne Inkubation bei 37°C sofort nach Zugabe der dsRNA zum Serum in Stickstoff schockgefroren. Die Lagerung erfolgte bei -80 °C.

### dsRNA-Isolierung:

Alle zur Isolierung eingesetzten Reagenzien mit Ausnahme einer Phenollösung wurden vor Gebrauch sterilfiltriert und auf Eis gekühlt.

Die bei -80 °C gelagerten Proben wurden auf Eis gestellt, mit je 450 µl einer 0,5 M Natriumchloridlösung versetzt und nach dem Auftauen für 5 Sekunden gründlich durchmischt.

Die Extraktion der dsRNA aus der Probenlösung erfolgte in Phase Lock-Gel-Reaktionsgefäßen (Eppendorf AG, 22331 Hamburg, Deutschland). Dazu wurden die Phase Lock-Gel-Reaktionsgefäße zunächst für 2 min bei 16 100 x g und 4°C zentrifugiert und auf Eis gestellt. Anschließend wurden die Proben in die Phase Lock-Gel-Reaktionsgefäße überführt und 500 µl Phenol:Chloroform:Isoamylalkohol-Mischung (Roti-Phenol, Carl Roth GmbH + Co., Schoemperlenstr. 1-5, D-76185 Karlsruhe, Deutschland) und 300 µl Chloroform zugesetzt. Die Proben wurden für 30 Sekunden mittels eines IKA Vibrax VXR basic, Typ VX2E (IKA Works do Brasil Ltda, Taquora, RJ 22713-000, Brasilien) gründlich durchmischt. Eine anschließende Phasentrennung erfolgte durch Zentrifugation bei 4°C und 16 100 x g für 15 min. Die obere wässrige Phase wurde vorsichtig in ein neues steriles Reaktionsgefäß überführt. Anschließend wurden 40 µl eisgekühlter 3 M Natriumacetat-Lösung (pH 5,2) zu der wässrigen Phase gegeben. Die resultierende Lösung wurde für 20 Sekunden gründlich gemischt. Nach Zugabe von 1 µl Pellet Paint (NF Co-Precipitant; Novagen, 441 Charmony Drive, Madison WI 53719, USA) wurde für 5 Sekunden gemischt. Anschließend wurde 1 ml eisgekühltes Ethanol zugegeben und für 20 Sekunden geschüttelt. Zur Präzipitation der dsRNA wurde die Lösung für eine Stunde auf -80°C gekühlt.

Die präzipitierte dsRNA wurde durch Zentrifugation bei 12 000 x g für 30 min bei 4°C pelletiert, dann der Überstand vorsichtig abgekippt und das Pellet mit 500 µl eisgekühltem 70% Ethanol (Mallinckrodt Baker B.V., 7400 AA. Deventer, Holland) gewaschen. Nach 2 Sekunden Schütteln, wurde erneut bei 12 000 x g und 4°C für 10 min zentrifugiert und der Überstand über der pelletierten dsRNA abgekippt. Die verbliebene Lösung wurde durch Zentrifugieren für 20 Sekunden bei 16 100 x g und 4°C am Gefäßboden gesammelt und abpipettiert. Die pelletierte dsRNA wurde bei geöffnetem Deckel und bei Raumtemperatur für 5 min getrocknet.

Die getrocknete dsRNA wurde in 30 µl Gelauftragungspuffer (95 % v/v Formamid, 10 mM EDTA, 0,025 % w/v Xylencyanol, 0,025 % w/v Bromphenolblau) durch 2-minütiges gründliches Mischen gelöst.

### Analyse durch denaturierende Gelelektrophorese:

Die Analyse der dsRNA erfolgte mittels denaturierender Polyacrylamid Gelelektrophorese in 0,8 mm dicken und 200 x 280 mm großen Gelen mit 8 M Harnstoff und 16 % v/v Formamid.

### Zusammensetzung eines Gels (50 ml):

- 24 g Harnstoff: (>99.5% p.a.; Carl Roth GmbH + Co., Schoemperlenstr. 1-5, D-76185 Karlsruhe, Deutschland),
- 18 ml Acrylamid: (rotiphorese Gel 29:1 (40%); Carl Roth GmbH + Co, Schoemperlenstr. 1-5, D-76185 Karlsruhe, Deutschland),
- 5 ml 10 X TBE: (1 M Tris (Ultra Qualität; Carl Roth GmbH + Co., Schoemperlenstr. 1-5, D-76185 Karlsruhe, Deutschland), 1M Borsäure (99,8% p.a.; Carl Roth GmbH + Co., Schoemperlenstr. 1-5, D-76185 Karlsruhe, Deutschland), 25 mM EDTA (Sigma-Aldrich Chemie GmbH P.O. 1120, D-89552 Steinheim, Deutschland) in entionisiertem Wasser),
- 8 ml Formamid: (Merck-Schuchardt, D-85662 Hohenbrunn, Deutschland),
- 50 µl Temed: (N,N,N',N'-Tetramethylethylendiamin) (Sigma-Aldrich Chemie GmbH P.O. 1120, D-89552 Steinheim, Deutschland) und
- 200 µl APS: Ammonium Persulfat (10 % w/v) (Gibco BRL Life Technologies, Invitrogen GmbH, Technologiepark Karlsruhe, Emmy-Noether-Str. 10, D-76131 Karlsruhe, Deutschland).

Nach dem Gießen des Gels zwischen zwei Glasplatten und dessen Auspolymerisieren für ca. 30 min wurde für 30 min bei 45 mA (Stromquelle: Power PAC 3000; Bio Rad Laboratories, 2000 Alfred Nobel Drive, Hercules, CA 94547, USA) ein Vorlauf in einer Gellaufapparatur durchgeführt. Als Gellaufpuffer wurde 1 x TBE verwendet. Zur gleichmäßigen Temperierung des Gels wurde eine 3 mm dicke Aluminiumplatte auf eine der Glasplatten geklemmt.

Die Proben wurden vor dem Auftrag aufs Gel für 5 min auf 100°C erhitzt, auf Eis abgeschreckt und für 20 Sekunden bei 13 000 x g und 4°C abzentrifugiert.

Von jeder Probe wurden 10 µl aufgetragen. Zusätzlich wurde eine nicht mit Serum inkubierte dsRNA-Probe (2 µl 25 µM dsRNA in 10 µl Gelauftragspuffer) aufgetragen.

Die Elektrophorese erfolgte für 90 min bei 45 mA. Abschließend wurde das Gel für 30 min mit Stains all (40 mg stains all (1-Ethyl-2-[3(3-Ethylnaphtho[1,2-d]Thiazolin-2-Ylidene)-2-Methylpropenyl] Naphtho-[1,2-d] Thiazolium Bromid); Sigma-Aldrich Chemie GmbH P.O. 1120, D-89552 Steinheim, Deutschland) + 400 ml Formamid (Merck-Schuchardt, D-85662 Hohenbrunn, Deutschland) + 400 ml H₂O) gefärbt, und dann für ca. 30 - 60 min im Wasserbad entfärbt. Die entfärbten Gele wurden mittels eines Fotodokumentationsgeräts (Image Master VDS Pharmacia Biotech; Amersham Biosciences Europe GmbH, Munzinger Str. 9, D-79111 Freiburg; by D & R, Israel) digitalisiert und zusätzlich im Farbmodus gescannt (Scanner: UMAX PowerLook 1100; UMAX Technologies, Inc., 10460 Brockwood Rd., Dallas, TX 75238 USA, Software: Adobe Photoshop Elements, Adobe Systems Incorporated, 345 Park Avenue, San Jose, California 95110-2704, USA).

### Ergebnisse:

Es sind die folgenden dsRNAs verwendet worden:
1. BCL20, deren Antisinn-Strang S1 zu einer Sequenz aus dem Sinn-Strang des humanen bcl-2-Gens (Datenbank GenBank, National Institutes of Health, Bethesda, Maryland, 20892, USA, Eintragungsnummer M13994) komplementär ist: 
S2: 5'-   GGC GAC UUC GCC GAG AUG UCC-3' (SEQ ID NO: 7)
S1: 3'-CG CCG CUG AAG CGG CUC UAC AGG-5' (SEQ ID NO: 8)
2. B133, deren Antisinn-Strang S1 zu dem Sinn-Strang des humanen bcl-2-Gens (GenBank, Eintragungsnummer M13994) komplementär ist: 
S2: 5'-   ACC GGG CAU CUU CUC CUC CCA-3' (SEQ ID NO: 9)
S1: 3'-CG UGG CCC GUA GAA GAG GAG GGU-5' (SEQ ID NO: 10[?]
3. P3, deren Antisinn-Strang S1 zum Sinn-Strang des humanen PLK1-Gens (GenBank, Eintragungsnummer X75932) komplementär ist:

S2: 5'-   GAU CAC CCU CCU UAA AUA UUU-3' (SEQ ID NO: 11)
S1: 3'-CG CUA GUG GGA GGA AUU UAU AAA-5' (SEQ ID NO: 12)

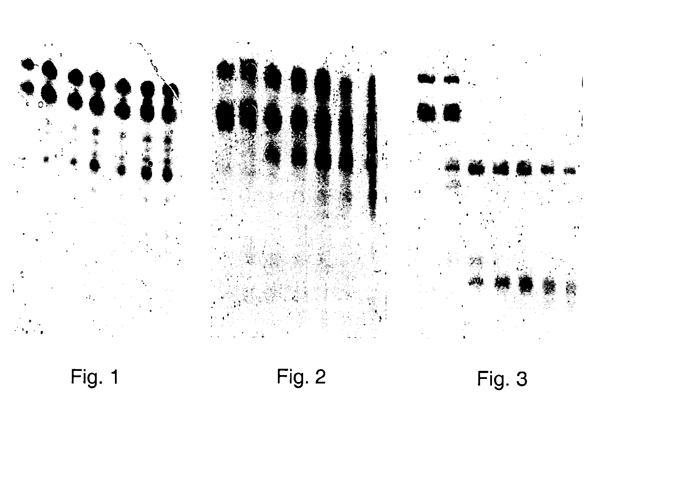

Fig. 1 bis 3 zeigen jeweils von links nach rechts eine gelelektrophoretische Auftrennung einer dsRNA ohne und nach 0, 15, 30, 60, 120 und 240 Minuten Inkubation in Serum. Fig. 1 zeigt die gelelektrophoretische Auftrennung der dsRNA BCL20,

Fig. 2 diejenige der dsRNA B133 und Fig. 3 diejenige der dsRNA P3.

Fig. 1 zeigt, dass die dsRNA BCL20 während der Inkubationszeit kaum abgebaut wird.

Aus Fig. 2 geht hervor, dass die dsRNA B133 etwas schneller abgebaut wird als die dsRNA BCL20. Das liegt daran, dass hier das jeweils letzte komplementäre Nukleotidpaar an beiden Enden der dsRNA nicht, wie es idealerweise der Fall ist, C-G ist.

Eine herkömmliche dsRNA, wie beispielsweise die in Fig. 3 dargestellte dsRNA P3, wird nahezu sofort in Serum abgebaut. Die dsRNA P3 weist nur an einem Ende der doppelsträngigen Struktur komplementäre G-C Nukleotidpaare auf.

## Patentansprüche

1. Verfahren zur gezielten Auswahl einer eine erhöhte Wirksamkeit aufweisenden doppelsträngigen aus zwei Einzelsträngen bestehenden Ribonukleinsäure (dsRNA) zur Hemmung der Expression eines Zielgens durch RNA-Interferenz, wobei die Sequenzen der Einzelstränge der dsRNA so gewählt werden, dass an beiden Enden der dsRNA jeweils das letzte komplementäre Nukleotidpaar G-C ist oder mindestens zwei der letzten vier komplementären Nukleotidpaare G-C-Paare sind, wobei die dsRNA an einem ersten Ende einen aus 1 bis 4 ungepaarten Nukleotiden gebildeten einzelsträngigen Überhang aufweist und an einem zweiten Ende keinen Überhang aufweist, wobei das sich direkt an das letzte komplementäre Nukleotidpaar anschließende ungepaarte Nukleotid des einzelsträngigen Übergangs eine Purin-Base enthält, wobei die folgenden dsRNAs ausgenommen sind: 
5'- CAG GAC CUC GCC GCU GCA GAC C-3' (SEQ ID NO: 1)
3'-CG GUC CUG GAG CGG CGA CGU CUG G-5' (SEQ ID NO: 2),
 
5'- G CCU UUG UGG AAC UGU ACG GCC-3' (SEQ ID NO: 3)
3'-UAC GGA AAC ACC UUG ACA UGC CGG-5' (SEQ ID NO: 4),
 
5'-CUU CUC CGC CUC ACA CCG CUG CAA-3' (SEQ ID NO: 5)
3'-GAA GAG GCG GAG UGU GGC GAC G  -5' (SEQ ID NO: 6),
 
5'- A CGG CUA GCU GUG AAA GGU CC-3' (SEQ ID NO: 13)
3'-AGU GCC GAU CGA CAC UUU CCA GG-5' (SEQ ID NO: 14) und
 und

5'-   CAA GGA GCA GGG ACA AGU UAC-3' (SEQ ID NO: 15)
3'-AA GUU CCU CGU CCC UGU UCA AUG-5' (SEQ ID NO: 16).

2. Verfahren nach Anspruch 1, wobei die Purin-Base G oder A ist.

3. Verfahren nach Anspruch 1 oder 2, wobei der Überhang aus 1 oder 2 ungepaarten Nukleotiden gebildet ist.

4. Verfahren nach einem der vorhergehenden Ansprüche, wobei der einzelsträngige Überhang zumindest zur Hälfte aus Purin-Basen, insbesondere G oder A, enthaltenden Nukleotiden gebildet ist.

5. Verfahren nach einem der vorhergehenden Ansprüche, wobei der Überhang, insbesondere ausschließlich, die Sequenz 5'-GC-3' aufweist.

6. Verfahren nach einem der vorhergehenden Ansprüche, wobei das erste Ende der dsRNA am 3'-Ende eines Antisinn-Strangs der dsRNA gelegen ist und das zweite Ende der dsRNA am 3'-Ende eines Sinn-Strangs der dsRNA gelegen ist.

7. Verfahren nach einem der vorhergehenden Ansprüche, wobei ein zum Zielgen komplementärer Bereich des Antisinn-Strangs der dsRNA 20 bis 23, insbesondere 22, Nukleotide aufweist.

8. Verfahren nach einem der vorhergehenden Ansprüche, wobei der Antisinn-Strang weniger als 30, vorzugsweise weniger als 25, besonders vorzugsweise 21 bis 24, Nukleotide aufweist.

9. Doppelsträngige aus zwei Einzelsträngen bestehende Ribonukleinsäure (dsRNA) zur Hemmung der Expression eines Zielgens durch RNA-Interferenz, wobei an beiden Enden der dsRNA jeweils das letzte komplementäre Nukleotidpaar G-C ist oder mindestens zwei der letzten vier komplementären Nukleotidpaare G-C-Paare sind, wobei die dsRNA an einem ersten Ende einen aus 1 bis 4 ungepaarten Nukleotiden gebildeten einzelsträngigen Überhang aufweist und an einem zweiten Ende keinen Überhang aufweist, wobei das sich direkt an das letzte komplementäre Nukleotidpaar anschließende ungepaarte Nukleotid des einzelsträngigen Übergangs eine Purin-Base enthält, wobei die folgenden dsRNAs ausgenommen sind: 
5'-   CAG GAC CUC GCC GCU GCA GAC C-3' (SEQ ID NO: 1)
3'-CG GUC CUG GAG CGG CGA CGU CUG G-5' (SEQ ID NO: 2),
 
5'- G CCU UUG UGG AAC UGU ACG GCC-3' (SEQ ID NO: 3)
3'-UAC GGA AAC ACC UUG ACA UGC CGG-5' (SEQ ID NO: 4),
 
5'-CUU CUC CGC CUC ACA CCG CUG CAA-3' (SEQ ID NO: 5)
3'-GAA GAG GCG GAG UGU GGC GAC G  -5' (SEQ ID NO: 6),
 
5'-  A CGG CUA GCU GUG AAA GGU CC-3'  (SEQ ID NO: 13)
3'-AGU GCC GAU CGA CAC UUU CCA GG-5'  (SEQ ID NO: 14)
 und

5'-   CAA GGA GCA GGG ACA AGU UAC-3' (SEQ ID NO: 15)
3'-AA GUU CCU CGU CCC UGU UCA AUG-5' (SEQ ID NO: 16).

10. DsRNA nach Anspruch 9, wobei die Purin-Base G oder A ist.

11. DsRNA nach Anspruch 9 oder 10, wobei der Überhang aus 1 oder 2 ungepaarten Nukleotiden gebildet ist.

12. DsRNA nach einem der Ansprüche 9 bis 11, wobei der einzelsträngige Überhang zumindest zur Hälfte aus Purin-Basen, insbesondere G oder A, enthaltenden Nukleotiden gebildet ist.

13. DsRNA nach einem der Ansprüche 9 bis 12, wobei der Überhang, insbesondere ausschließlich, die Sequenz 5'-GC-3' aufweist.

14. DsRNA nach einem der Ansprüche 9 bis 13, wobei das erste Ende der dsRNA am 3'-Ende eines Antisinn-Strangs der dsRNA und das zweite Ende der dsRNA am 3'-Ende eines Sinn-Strangs der dsRNA gelegen ist.

15. DsRNA nach einem der Ansprüche 9 bis 14, wobei ein zum Zielgen komplementärer Bereich des Antisinn-Strangs der dsRNA 20 bis 23, insbesondere 22, Nukleotide aufweist.

16. DsRNA nach einem der Ansprüche 9 bis 15, wobei der Antisinn-Strang weniger als 30, vorzugsweise weniger als 25, besonders vorzugsweise 21 bis 24, Nukleotide aufweist.

17. Verwendung einer dsRNA nach einem der Ansprüche 9 bis 16 zur Hemmung der Expression eines Zielgens durch RNA-Interferenz.

18. Medikament zur Hemmung der Expression eines Zielgens durch RNA-Interferenz, wobei das Medikament eine doppelsträngige aus zwei Einzelsträngen bestehende Ribonukleinsäure (dsRNA) nach einem der Ansprüche 9 bis 16 enthält.
